# FASCICULE DE BREVET EUROPEEN

(11) **EP 0 566 190 B1**
(45) Date de publication et mention de la délivrance du brevet: **04.09.1996**
(21) Numéro de dépôt: 93201013.5
(22) Date de dépôt: 06.04.1993
(51) Int. Cl.: C07C 17/38, C07C 19/08

(54) **Procédé pour l'épuration de 1,1-dichloro-l-fluoroéthane**
Verfahren zur Reinigung von 1,1-Dichlor-1-Fluorethan
Process for the purification of 1,1-dichloro-1-fluoroethane

(30) Priorité: 17.04.1992 BE 9200357
(43) Date de publication de la demande: 20.10.1993
(73) Titulaire: SOLVAY (Société Anonyme), B-1050 Bruxelles (BE)
(72) Inventeur: Walraevens, René, B-1080 Bruxelles (BE); Janssens, Francine, B-1800 Vilvoorde (BE); Catinat, Jean-Pierre, B-7130 Binche (BE)
(74) Mandataire: Jacques, Philippe

(56) Documents cités:
- EP-A- 0 401 493
- EP-A- 0 420 709
- GB-A- 1 401 541

## Description

La présente invention concerne un procédé perfectionné pour l'épuration de 1,1-dichloro-1-fluoroéthane brut par traitement avec du chlore suivi de distillation.

Le 1,1-dichloro-1-fluoroéthane (HFA-141b) est un hydrocarbure chlorofluoré partiellement halogéné qui s'avère être un substitut intéressant de certains hydrocarbures chlorofluorés entièrement halogénés (CFC) dont la production et l'utilisation sont progressivement réduites parce qu'ils sont suspectés d'avoir un effet néfaste sur la couche d'ozone.

Le 1,1-dichloro-1-fluoroéthane brut des procédés de synthèse est généralement contaminé par des sous-produits de la synthèse et des impuretés indésirables. Certains de ces sous-produits et impuretés peuvent être aisément séparés par distillation. C'est notamment le cas pour le 1-chloro-1,1-difluoroéthane, le 1,1,1-trifluoroéthane ou le 1,1,1-trichloroéthane, ainsi que pour les composés plus lourds comprenant un plus grand nombre d'atomes de carbone, formés en cours de réaction. Toutefois, le 1,1-dichloro-1-fluoroéthane contient généralement en tant qu'impuretés de petites quantités de composés insaturés chlorés et/ou chlorofluorés dont la séparation par distillation s'avère difficile, vu leur point d'ébullition voisin de celui du 1,1-dichloro-1-fluoroéthane.

Outre le chlorure de vinylidène, qui constitue l'impureté la plus importante, les impuretés insaturées qui peuvent être présentes dans le 1,1-dichloro-1-fluoroéthane à purifier sont principalement les 1,2-dichlorofluoroéthylènes cis et trans, le 1,2-dichloroéthylène trans, le dichloroacétylène et le 1-chloro-1-fluoroéthylène.

La demande de brevet EP-A-0401493 de ATOCHEM NORTH AMERICA décrit la purification de 1,1-dichloro-1-fluoroéthane par chloration photochimique des impuretés insaturées suivie d'une séparation par distillation. Ce procédé est mis en oeuvre à une température telle que le 1,1-dichloro-1-fluoroéthane soit en phase liquide sous la pression opératoire. Les conditions opératoires sont aussi en partie limitées par la présence de la source d'énergie lumineuse.

L'accent est mis dans ce document sur l'épuration en chlorure de vinylidène mais dans la plupart des exemples, il n'a pas été possible de descendre en-dessous de 120 ppm en poids de ce composé. En outre, il n'est pas fait mention de l'élimination des 1,2-dichlorofluoroéthylènes cis et trans, impuretés plus halogénées dont la conversion apparaît plus difficile, d'autant que dans certains cas leur concentration peut, après être passée par un minimum, croître de nouveau. A titre de référence, signalons que les spécifications PAFT II (Program for Alternative Fluorocarbon Toxicity testing) imposent une teneur totale en impuretés insaturées chlorofluorées ne dépassant pas 10 ppm en poids.

La présente invention a dès lors pour but de procurer un procédé perfectionné pour l'épuration de 1,1-dichloro-1-fluoroéthane brut par traitement avec du chlore suivi d'une distillation qui permette non seulement une meilleure épuration en chlorure de vinylidène mais également une séparation efficace et rapide des autres impuretés insaturées, notamment les 1,2-dichlorofluoroéthylènes cis et trans.

A cet effet, l'invention concerne un procédé pour l'épuration de 1,1-dichloro-1-fluoroéthane brut par traitement avec du chlore puis distillation, le traitement avec le chlore étant réalisé en présence d'un initiateur organique de radicaux libres.

Dans le procédé selon l'invention, on entend désigner par 1,1-dichloro-1-fluoroéthane brut le 1,1-dichloro-1-fluoroéthane et les impuretés (en ce compris les sous-produits de sa fabrication) qu'il contient.

Pour favoriser le mélange du 1,1-dichloro-1-fluoroéthane brut avec l'initiateur organique, le procédé selon l'invention est de préférence réalisé en phase liquide.

L'initiateur organique de radicaux libres a pour fonction de décomposer les molécules de chlore par rupture radicalaire. Selon l'invention, l'initiateur de radicaux libres est un composé organique. Parmi les composés organiques, on utilise le plus souvent les composés peroxydés ou diazoïques. On met en particulier en oeuvre les composés peroxydés. Parmi ceux-ci, on choisit plus particulièrement les peroxydes de diacyle, peroxydicarbonates, peresters d'alkyle, peracétals, peroxydes de cétone, hydroperoxydes d'alkyle ou peroxydes de dialkyle. On retient de préférence les peroxydes de diacyle ou les peroxydicarbonates. D'excellents résultats ont été obtenus avec le peroxyde de dilauroyle, le peroxyde de dibenzoyle ou le peroxydicarbonate de dicétyle. L'initiateur organique est de préférence sélectionné parmi les composés présentant une durée de demi-vie de 0,1 à 3 heures et, le plus souvent, d'environ 1 heure à la température du traitement avec le chlore.

L'initiateur organique peut être mis en oeuvre à des doses très variables. Il est généralement utilisé à raison d'au moins 10 ppm en poids par rapport au 1,1-dichloro-1-fluoroéthane brut. On met en particulier en oeuvre au moins environ 20 ppm en poids d'initiateur organique et plus particulièrement encore au moins environ 30 ppm en poids. Le plus souvent, on n'utilise pas plus d'environ 10.000 ppm en poids d'initiateur organique par rapport au 1,1-dichloro-1-fluoroéthane brut. De préférence on ne dépasse pas environ 1.000 ppm en poids d'initiateur organique et plus préférentiellement encore on ne dépasse pas environ 300 ppm en poids.

Le traitement avec le chlore a pour fonction de chlorer les impuretés insaturées du 1,1-dichloro-1-fluoroéthane brut. Il a notamment pour fonction de convertir le chlorure de vinylidène, les 1,2-dichlorofluoroéthylènes cis et trans et le dichloroacétylène.

Le chlore peut être mis en oeuvre en phase gazeuse ou en phase liquide. Il est introduit en quantités excédentaires par rapport à l'ensemble des impuretés insaturées à chlorer dans le 1,1-dichloro-1-fluoroéthane brut. Généralement, le chlore est mis en oeuvre à raison de plus de 3 moles par mole d'impuretés insaturées, de préférence au moins environ 4 moles par mole d'impuretés insaturées. Le plus souvent, il n'est pas souhaitable de dépasser environ 40 moles de chlore par mole d'impuretés insaturées. Il est préférable de limiter la quantité mise en oeuvre afin que quasiment tout le chlore puisse réagir et ne se retrouve pas comme tel en aval du présent traitement d'épuration. De préférence, on ne dépasse pas 15 moles par mole d'impuretés insaturées et plus préférentiellement encore ce rapport n'excède pas environ 12.

Le traitement avec du chlore peut être réalisé dans une large gamme de températures. En particulier, le traitement avec du chlore est réalisé à une température d'au moins 40 °C et plus particulièrement encore de plus d'environ 60 °C. Des températures plus élevées permettent une transformation plus rapide des composés insaturés, plus particulièrement des 1,2-dichlorofluoroéthylènes, sans formation trop importante de composés lourds pouvant résulter de réactions parallèles de chloration substitutive. Il en résulte cependant un accroissement corrélatif de la pression qu'il convient de prendre en compte. De préférence, la température de traitement ne dépasse pas 150 °C et plus préférentiellement encore, elle ne dépasse pas environ 100 °C. D'excellents résultats ont été obtenus lorsque le traitement avec du chlore est réalisé aux environs de 60 à 100 °C.

Le traitement avec du chlore peut être réalisé à la pression autogène ou une pression supérieure générée par l'introduction d'un gaz inerte. En général, le traitement est réalisé à une pression qui ne dépasse pas environ 5 MPa, de préférence 2 MPa. Des pressions d'environ 0,2 à environ 1,0 MPa conviennent bien.

Ces conditions corrélées de pression et de température élevées permises pour le traitement avec du chlore contribuent à l'élimination efficace et rapide des impuretés insaturées.

La durée du traitement avec du chlore peut être d'environ 1 à environ 120 minutes. De préférence, la durée du traitement avec du chlore est au maximum de 60 minutes.

En présence d'un important excès de chlore, on adaptera la durée du traitement pour limiter les pertes en 1,1-dichloro-1-fluoroéthane par chloration substitutive et formation de 1,1,2-trichloro-1-fluoroéthane.

On limitera également la présence d'ions métalliques, qui pourraient être à l'origine de la reformation des 1,2-dichlorofluoroéthylènes cis et trans, par déshydrochloration du 1,1,2-trichloro-1-fluoroéthane susmentionné. Cette explication ne lie cependant pas la demanderesse.

Le réacteur de chloration et les appareils de distillation sont dès lors de préférence réalisés avec des matériaux résistant à la corrosion, tels que notamment les alliages de type MONEL, INCONEL ou HASTELLOY.

Pendant le traitement avec le chlore, on veille à ce que la teneur en oxygène dans le chlore soit inférieure à 1000 ppm en volume et de préférence qu'elle ne dépasse pas 50 ppm en volume. Pour ce faire, le 1,1-dichloro-1-fluoroéthane brut est d'abord désaéré par barbotage avec un gaz inerte, de l'azote par exemple.

La distillation qui suit le traitement avec le chlore a pour fonction de séparer les impuretés du 1,1-dichloro-1-fluoroéthane, après leur chloration. La distillation peut être réalisée par tous moyens classiques connus.

Suivant une variante de réalisation avantageuse du procédé selon l'invention, on introduit l'initiateur organique dans le 1,1-dichloro-1-fluoroéthane brut avant le chlore. Dans une variante d'exécution préférée de cette forme de réalisation de l'invention, on introduit le chlore dans le 1,1-dichloro-1-fluoroéthane à une température voisine de celle du traitement. Dans une variante d'exécution particulièrement préférée de cette forme de réalisation de l'invention, on introduit également l'initiateur organique dans le 1,1-dichloro-1-fluoroéthane à une température voisine de celle du traitement.

Le procédé selon l'invention s'applique à l'épuration de 1,1-dichloro-1-fluoroéthane brut préparé par tout procédé de synthèse, sans qu'un traitement préalable ne soit requis. Le procédé selon l'invention trouve une application intéressante à l'épuration de 1,1-dichloro-1-fluoroéthane obtenu par synthèse du chlorure de vinylidène et du fluorure d'hydrogène. Le procédé selon l'invention peut notamment être mis en oeuvre en présence de composés dont le point d'ébullition est sensiblement supérieur à celui du 1,1-dichloro-1-fluoroéthane. De préférence, on sépare cependant préalablement ces composés du 1,1-dichloro-1-fluoroéthane. Il est également préférable de séparer préalablement les composés dont le point d'ébullition est sensiblement inférieur à celui du 1,1-dichloro-1-fluoroéthane, tels que notamment le 1-chloro-1,1-difluoroéthane et le 1,1,1-trifluoroéthane. Ces séparations peuvent être réalisées de manière classique par distillation.

Les exemples 1 à 3 illustrent l'invention de manière non limitative. L'exemple 4R est donné à titre de référence.

### Exemples

### Exemple 1

Dans un autoclave en alliage HASTELLOY de 0,5 l, équipé d'un agitateur, préalablement refroidi à 0 °C et mis sous vide de 1 500 Pa, on a introduit, par aspiration, 300 ml de 1,1-dichloro-1-fluoroéthane brut, contenant 110 ppm en poids de peroxyde de dilauroyle (temps de demi-vie de 1 h à 80 °C). La solution, maintenue à 0 °C, a ensuite été désaérée par des passages répétés d'azote.

Le mélange réactionnel a alors été amené à 76 °C en plongeant le réacteur dans un bain thermostatisé préchauffé. La pression était à ce stade de 4,2.10⁵ Pa.

On a introduit ensuite 3 g de chlore, soit 5,2 moles de chlore par mole d'impuretés insaturées. La température est passée à 81 °C et a été maintenue à cette valeur tandis que la pression autogène a augmenté pour atteindre 5,2.10⁵ Pa après 23 minutes.

Des échantillons ont été prélevés en cours d'essai, à l'aide d'un tube plongeant. Ils ont été directement recueillis dans une ampoule contenant 50 ml d'une solution aqueuse saturée en bicarbonate de soude, préalablement refroidie dans la glace. Après décantation et séchage sur CaCl₂, la phase organique recueillie a été analysée par chromatographie en phase gazeuse.

Le **tableau 1** reprend les teneurs en impuretés insaturées en mg.kg-¹ dans le 1,1-dichloro-1-fluoroéthane brut, avant introduction du chlore, puis 23 minutes après l'introduction de chlore.

**TABLEAU 1**

| Composé insaturé | Mélange réactionnel initial | après traitement t = 23 min |
|---|---|---|
| Chlorure de vinylidène | 850 | <2 |
| Dichloroacétylène | 228 | <1 |
| 1,2-dichloroéthylène trans | 920 | <1 |
| 1,2-dichlorofluoroéthylène cis | 79 | 1 |
| 1,2-dichlorofluoroéthylène trans | 43 | <2 |
| 1-chloro-1-fluoroéthylène | 2 | <1 |

Ces résultats illustrent qu'après seulement 23 minutes de traitement, il ne reste que moins de 2 ppm en poids de chacune des impuretés insaturées présentes, notamment de chlorure de vinylidène et des 1,2-dichlorofluoroéthylènes cis et trans. On peut alors par distillation obtenir du 1,1-dichloro-1-fluoroéthane de grande pureté.

### Exemple 2

Dans un autoclave identique à celui de l'exemple 1, on a introduit de la même manière 300 ml de 1,1-dichloro-1-fluoroéthane brut, contenant 71 ppm en poids de peroxyde de dibenzoyle (durée de demi-vie de 1 h à 91 °C). La solution, maintenue à 0 °C, a ensuite été désaérée par des passages répétés d'azote.

Le mélange réactionnel a alors été amené à 92 °C en plongeant le réacteur dans un bain thermostatisé préchauffé. La pression était à ce stade de 6,3.10⁵ Pa.

On a introduit ensuite 4,8 g de chlore, soit 8 moles de chlore par mole d'impuretés insaturées. La température est passée à 96 °C et a été maintenue à cette valeur tandis que la pression autogène a augmenté pour atteindre 7.10⁵ Pa après 15 minutes.

Des échantillons ont été prélevés en cours d'essai et analysés comme dans l'exemple 1.

Le **tableau 2** reprend les teneurs en impuretés insaturées chlorées et chlorofluorées en mg.kg⁻¹ dans le 1,1-dichloro-1-fluoroéthane brut, avant introduction du chlore, puis 15 minutes après l'introduction de chlore.

**TABLEAU 2**

| Composé insaturé | Mélange réactionnel initial | après traitement t = 15 min |
|---|---|---|
| Chlorure de vinylidène | 863 | 2 |
| Dichloroacétylène | 230 | <1 |
| 1,2-dichloroéthylène trans | 937 | <1 |
| 1,2-dichlorofluoroéthylène cis | 80 | 1 |
| 1,2-dichlorofluoroéthylène trans | 42 | <1 |
| 1-chloro-1-fluoroéthylène | 2 | 1 |

### Exemple 3

Dans un autoclave identique à celui de l'exemple 1, on a introduit de la même manière 300 ml de 1,1-dichloro-1-fluoroéthane brut, contenant 156 ppm en poids de peroxydicarbonate de dicétyle (durée de demi-vie de 1 h à 57 °C). La solution, maintenue à 0 °C, a ensuite été désaérée par des passages répétés d'azote.

Le mélange réactionnel a alors été amené à 57 °C en plongeant le réacteur dans un bain thermostatisé préchauffé. La pression était à ce stade de 2,9.10⁵ Pa.

On a introduit ensuite 4,8 g de chlore, soit 11 moles de chlore par mole d'impuretés insaturées. La température est passée à 63 °C et a été maintenue à cette valeur tandis que la pression autogène a augmenté pour atteindre 3,8.10⁵ Pa après 22 minutes.

Des échantillons ont été prélevés en cours d'essai et analysés comme dans l'exemple 1.

Le **tableau 3** reprend les teneurs en impuretés insaturées chlorées et chlorofluorées en mg.kg⁻¹ dans le 1,1-dichloro-1-fluoroéthane brut, avant introduction du chlore, puis 22 minutes après l'introduction de chlore.

**TABLEAU 3**

| Composé insaturé | Mélange réactionnel initial | après traitement t = 22 min |
|---|---|---|
| Chlorure de vinylidène | 879 | <1 |
| Dichloroacétylène | 291 | <1 |
| 1,2-dichloroéthylène trans | 945 | 3 |
| 1,2-dichlorofluoroéthylène cis | 84 | 1 |
| 1,2-dichlorofluoroéthylène trans | 47 | <1 |
| 1-chloro-1-fluoroéthylène | 1 | <1 |

### Exemple 4R (de référence)

Dans un flacon de 10 ml, on a introduit à température ambiante 10 g de 1,1-dichloro-1-fluoroéthane brut. On a ensuite introduit en une seule fois, à l'aide d'une seringue, au travers du bouchon téflonné fermant le flacon, une quantité de chlore gazeux correspondant à 3 moles de chlore par mole d'impuretés insaturées.
L'échantillon a alors été exposé au rayonnement d'une lampe UV Philips HP 80, dans une enceinte fermée (distance lampe - échantillon : 15 cm). La température a été maintenue à environ 35 °C par circulation d'air dans l'enceinte.

L'échantillon a ensuite été analysé par chromatographie en phase gazeuse.

Le **tableau 4** reprend les teneurs en impuretés insaturées chlorées et chlorofluorées en mg.kg⁻¹ dans le 1,1-dichloro-1-fluoro-éthane brut, avant introduction du chlore, puis 2,5 h et 8 h après introduction du chlore.

**TABLEAU 4**

| Composé insaturé | Mélange réactionnel initial | après traitement | |
|---|---|---|---|
| | | t= 2,5 h | t= 8 h |
| Chlorure de vinylidène | 295 | 3 | <1 |
| Dichloroacétylène | 6 | <1 | <1 |
| 1,2-dichloroéthylène trans | 189 | 5 | 13 |
| 1,2-dichlorofluoroéthylène cis | 110 | 5 | 7 |
| 1,2-dichlorofluoroéthylène trans | 114 | 9 | 10 |
| 1-chloro-1-fluoroéthylène | 12 | 2 | 3 |

Cet exemple de référence illustre que la chloration complète du chlorure de vinylidène par voie photochimique exige un très long temps de traitement et que, même à ce moment, les 1,2-dichlorofluoroéthylènes cis et trans et le 1-chloro-1-fluoroéthylène restent présents en quantités importantes par rapport aux résultats obtenus avec le procédé selon la présente invention.

## Revendications

1. Procédé pour l'épuration de 1,1-dichloro-1-fluoroéthane brut par traitement avec du chlore puis distillation, caractérisé en ce que le traitement avec le chlore est réalisé en présence d'un initiateur organique de radicaux libres.

2. Procédé selon la revendication 1, caractérisé en ce que l'initiateur organique est choisi parmi les composés peroxydés ou diazoïques.

3. Procédé selon la revendication 2, caractérisé en ce que l'initiateur organique est choisi parmi les composés peroxydés.

4. Procédé selon la revendication 3, caractérisé en ce que le composé peroxydé est choisi parmi les peroxydes de diacyle et les peroxydicarbonates.

5. Procédé selon la revendication 4, caractérisé en ce que le composé peroxydé est choisi parmi le peroxyde de dilauroyle, le peroxyde de dibenzoyle et le peroxydicarbonate de dicétyle.

6. Procédé selon l'une quelconque des revendications précédentes, caractérisé en ce qu'on sélectionne l'initiateur organique parmi les composés présentant une durée de demi-vie d'environ 1 heure à la température du traitement avec le chlore.

7. Procédé selon l'une quelconque des revendications précédentes, caractérisé en ce que le traitement avec le chlore est réalisé en présence d'au moins 10 ppm en poids d'initiateur organique par rapport au poids de 1,1-dichloro-1-fluoroéthane brut.

8. Procédé selon l'une quelconque des revendications précédentes, caractérisé en ce que le chlore est mis en oeuvre en un rapport de plus de 3 moles sans dépasser 15 moles par mole d'impuretés insaturées dans le 1,1-dichloro-1-fluoroéthane brut.

9. Procédé selon l'une quelconque des revendications précédentes, caractérisé en ce que le traitement avec le chlore est réalisé à une température de 40 à 150 °C.

10. Procédé selon l'une quelconque des revendications précédentes, caractérisé en ce que la durée du traitement avec le chlore est au maximum de 60 minutes.

## Claims

1. Process for the purification of crude 1,1-dichloro-1-fluoroethane by treatment with chlorine and then distillation, characterised in that the treatment with chlorine is carried out in the presence of an organic free radical initiator.

2. Process according to Claim 1, characterised in that the organic initiator is chosen from the peroxide or diazo compounds.

3. Process according to Claim 2, characterised in that the organic initiator is chosen from the peroxide compounds.

4. Process according to Claim 3, characterised in that the peroxide compound is chosen from the diacyl peroxides and the peroxydicarbonates.

5. Process according to Claim 4, characterised in that the peroxide compound is chosen from dilauroyl peroxide, dibenzoyl peroxide and dicetyl peroxydicarbonate.

6. Process according to any one of the preceding claims, characterised in that the organic initiator is selected from the compounds exhibiting a half-life of approximately 1 hour at the temperature of the treatment with chlorine.

7. Process according to any one of the preceding claims, characterised in that the treatment with chlorine is carried out in the presence of at least 10 ppm by weight of organic initiator with respect to the weight of crude 1,1-dichloro-1-fluoroethane.

8. Process according to any one of the preceding claims, characterised in that the chlorine is used in a ratio of more than 3 mol without exceeding 15 mol per mole of unsaturated impurities in the crude 1,1-dichloro-1-fluoroethane.

9. Process according to any one of the preceding claims, characterised in that the treatment with chlorine is carried out at a temperature of 40 to 150°C.

10. Process according to any one of the preceding claims, characterised in that the duration of the treatment with chlorine is at most 60 minutes.

## Patentansprüche

1. Verfahren zur Reinigung von rohem 1,1-Dichlor-1-fluorethan durch Behandlung mit Chlor und anschließender Destillation, dadurch gekennzeichnet, daß die Behandlung mit Chlor in Gegenwart eines organischen Initiators von freien Radikalen durchgeführt wird.

2. Verfahren gemäß Anspruch 1, dadurch gekennzeichnet, daß der organische Initiator unter den Peroxid- oder Diazoverbindungen ausgewählt ist.

3. Verfahren gemäß Anspruch 2, dadurch gekennzeichnet, daß der organische Initiator unter den Peroxidverbindungen ausgewählt ist.

4. Verfahren gemäß Anspruch 3, dadurch gekennzeichnet, daß die Peroxidverbindung unter den Diacylperoxiden und den Peroxydicarbonaten ausgewählt ist.

5. Verfahren gemäß Anspruch 4, dadurch gekennzeichnet, daß die Peroxidverbindung unter Dilauroylperoxid, Dibenzoylperoxid und Diketylperoxydicarbonat ausgewählt ist.

6. Verfahren gemäß einem der vorangehenden Ansprüche, dadurch gekennzeichnet, daß man den organischen Initiator unter den Verbindungen auswählt, die bei der Temperatur der Chlorbehandlung eine Halbwertszeit von ungefähr einer Stunde aufweisen.

7. Verfahren gemäß einem der vorangehenden Ansprüche, dadurch gekennzeichnet, daß die Behandlung mit Chlor in Gegenwart von wenigstens 10 Gew.-ppm organischem Initiator bezogen auf das Gewicht des rohen 1,1-Dichlor-1-fluorethans durchgeführt wird.

8. Verfahren gemäß einem der vorangehenden Ansprüche, dadurch gekennzeichnet, daß das Chlor in einem Verhältnis von mehr als 3 Mol, ohne 15 Mol zu überschreiten, pro Mol ungesättigter Verunreinigungen in dem rohen 1,1-Dichlor-1-fluorethan eingesetzt wird.

9. Verfahren gemäß einem der vorangehenden Ansprüche, dadurch gekennzeichnet, daß die Behandlung mit Chlor bei einer Temperatur von 40 bis 150 °C durchgeführt wird.

10. Verfahren gemäß einem der vorangehenden Ansprüche, dadurch gekennzeichnet, daß die Dauer der Behandlung mit Chlor maximal 60 Minuten beträgt.
